(19) Europäisches Patentamt

European Patent Office

Office européen des brevets

(11) EP 1 704 919 A1

(12) EUROPEAN PATENT APPLICATION

(43) Date of publication:
27.09.2006 Bulletin 2006/39

(51) Int Cl.:
$B01J\ 23/00\ ^{(2006.01)}$    $B01J\ 23/28\ ^{(2006.01)}$
$B01J\ 23/20\ ^{(2006.01)}$    $B01J\ 23/18\ ^{(2006.01)}$
$B01J\ 37/02\ ^{(2006.01)}$    $B01J\ 37/08\ ^{(2006.01)}$
$C07C\ 51/215\ ^{(2006.01)}$    $C07C\ 57/04\ ^{(2006.01)}$

(21) Application number: 05290665.8

(22) Date of filing: 25.03.2005

(84) Designated Contracting States:
AT BE BG CH CY CZ DE DK EE ES FI FR GB GR
HU IE IS IT LI LT LU MC NL PL PT RO SE SI SK TR
Designated Extension States:
AL BA HR LV MK YU

(71) Applicant: Arkema France
92800 Puteaux (FR)

(72) Inventors:
• Dubois, Jean-Luc
69390 Millery (FR)
• Ueda, Wataru
Tokyo 194-0044 (JP)
• Endo, Yusuke
Sapporo-shi
Hokkaido 001-0020 (JP)

(54) **Process for preparing improved catalysts for selective oxidation of propane into acrylic acid**

(57) The invention concerns the selective oxidation of propane into acrylic acid using highly selective catalysts and relates more particularly to a process for preparing these improved catalysts and their use for the production of acrylic acid from propane.

The improved catalysts having the formula : $Mo_1V_a$ (Te and/or Sb)$_b$(Nb and/or Ta)$_c$Si$_d$O$_x$

Wherein
a is between 0.006 and 1
b is between 0.006 and 1
c is between 0.001 and 0.5
d is between 0 and 3.5
and x is dependant on the oxidation state of the other elements,

are prepared by adding to a mainly orthorhombic phase of a Mo-V-(Te and/or Sb)-O mixed metal oxide, optionally containing Nb and/or Ta and/or Si, a doping agent constituted by at least one component selected from Nb and Ta.

EP 1 704 919 A1

Printed by Jouve, 75001 PARIS (FR)

**Description**

The invention concerns the selective oxidation of propane into acrylic acid using highly selective catalysts and relates more particularly to a process for preparing these improved catalysts and their use for the production of acrylic acid from propane.

The selective oxidation of light alkanes into oxygenated products is a very attractive way for the chemical utilization of large natural gas resources. Nowadays, there is an increasing interest in the development of a process for direct oxidation of propane to acrylic acid as an alternative to the two-step conventional industrial process. In this selective oxidation field, catalysts performances relating to the conversion of propane and selectivity to acrylic acid are the most often too limited; and there is a need to improve catalytic performances of solids generally used for this reaction.

The patent application EP-A-608838 describes catalysts containing a mixed metal oxide comprising as essential components, Mo, V, Te, O and X wherein X is at least one element selected from the group consisting of niobium, tantalum, tungsten, titanium, aluminium, zirconium, chromium, manganese, iron, ruthenium, cobalt, rhodium, nickel, palladium, platinum, antimony, bismuth, boron, indium and cerium, these elements being present in determined proportions. When a MoVTeNbO mixed metal oxide is to be prepared, an aqueous solution of telluric acid, an aqueous solution of ammonium niobium oxalate and a solution or slurry of ammonium paramolybdate are sequentially added to an aqueous solution containing ammonium metavanadate, the mixture in then dried and finally the remaining dried product is calcined. Preferred is the catalyst which exhibits the main peaks at $2\theta = 22.1$ ° and $28.2°$ in the X-ray diffraction pattern. This catalyst is claimed to achieve acrylic acid yields substantially better than the conventional methods. With a comparative MoVTeO mixed metal oxide, no formation of acrylic acid was detected.

MoVMO catalysts (M=Al, Ga, Bi, Sb and Te), prepared by hydrothermal synthesis, have also been studied in the partial oxidation of propane (Applied Catalysis A200 (2000) 135-143). However, MoVSbO catalysts present selectivity to acid acrylic lower than that obtained on Te-based catalyst, although their catalyst performance has partially been enhanced by grinding the catalyst after calcination step.

Japanese Laid-Open Patent application Publication N°10-330343 discloses catalysts useful for production of nitriles by vapour phase oxidation of an alkane. These catalysts having a crystalline structure are represented by the formula $MO_aV_bSb_cX_xO_n$ wherein X is one or more kinds of metallic elements selected from Ti, Zr, Nb, Ta, Cr....A precursor is first prepared by addition of solutions or suspensions containing respectively a source of antimony and a source of vanadium, then addition of a solution or suspension containing a specific amount of molybdenum and addition of the element X as powder form or solution. This precursor is then dried and calcined. The solid obtained is a mixed metal oxide having specific main and powdery X-ray diffraction peaks corresponding to a mixture of an orthorhombic phase and hexagonal phase material. Further treatments as the washing with a solvent selected from aqueous oxalic acid, ethylene glycol or aqueous hydrogen peroxide, allow separating the orthorhombic phase material as an improved catalyst.

It is known by US patent 6,060,422 to use a metal oxide containing metallic elements Mo, V, Sb and A, wherein A can be Nb or Ta for use in producing acrylic acid by the gas-phase catalytic oxidation of propane. The process for producing this catalyst comprises a step (1) of reacting $V^{+5}$ with $Sb^{+3}$ in an aqueous medium at a temperature not lower than 70°C in the presence of $Mo^{+6}$ and, during or after the reaction, bubbling either molecular oxygen or a gas containing molecular oxygen into the reaction mixture, and a step (2) of adding a compound containing the element A to the reaction product obtained in step (1), mixing the ingredients to obtain a homogeneous mixture, and calcining the resultant mixture. In this process the metal A is added to either the dispersion which is the reaction mixture resulting from the above reaction and contains Mo, V and Sb, or a solid matter obtained by subjecting the dispersion to evaporation to dryness. The metal oxide obtained by this process reveals peak at a diffraction angle 2 θ of 28.1. In the catalytic production of acrylic acid from propane, selectivity for acrylic acid of 29.5% is obtained at a reaction temperature of 400°C. Higher selectivity can be performed while using as catalyst a metal oxide obtained by depositing at least one compound which contains an element B selected from the group consisting of Na, K, Rb, Cs, P and As on the oxide obtained above.

In US patent application 2003/0013904, there are provided catalysts wherein the performance for the vapour phase oxidation of an alkane to an unsaturated carboxylic acid is enhanced by doping catalysts comprising a mixed metal oxide which can be a MoV(Te or Sb)(Nb or Ta) mixed metal oxide, with a metal or a combination of metals. The preferred dopants are Pd or Pd-Au alloys. In a first step, a catalyst precursor admixture is formed by admixing metal compounds and at least one solvent to form the admixture that may be a slurry, solution or combination thereof. Liquids are then removed and the resulting precursor admixture is calcined. The dopants are introduced prior to, during or after calcination by sputtering.

In US patent application 2002/01831198, a mixed metal oxide, which may be an orthorhombic phase material, is improved as a catalyst for the production of unsaturated carboxylic acids from alkanes, by a process comprising contacting with a liquid contact member selected from the group consisting of organic acids, alcohols, inorganic acids and hydrogen peroxide.

The prior art continues to seek ways to improve the performances of mixed metal oxide catalysts for the production of acrylic acid from propane.

It has now been found that the performance of a Mo-V-(Te and/or Sb)-(Nb and/or Ta)-O mixed metal oxide catalyst having the formula (I) :

$$Mo_1V_a(Te\text{ and/or }Sb)_b(Nb\text{ and/or }Ta)_cSi_dO_x \qquad (I)$$

Wherein

a is between 0.006 and 1
b is between 0.006 and 1
c is between 0.001 and 0.5
d is between 0 and 3.5
and x is dependent on the oxidation state of the other elements,

may be improved for the oxidation of propane to acrylic acid by carrying out a new process for its preparation. This process comprises addition of a doping agent, constituted by at least one component selected from niobium and tantalum to a Mo-V-Sb (and/or Te)-O catalyst, optionally containing Nb and/or Ta and/or Si. The addition of the doping agent increases the number of Nb and/or Ta sites on the surface of the catalyst and changes the catalyst properties. It is well known that Nb or Ta have a predominant function with regard to selectivity of the oxidation of propane into acrylic acid. When the complete amount of Nb or Ta is introduced during the synthesis, it's difficult to control the Nb or Ta amount present at the surface of the solid. The niobium or tantalum doping allows controlling the amount of Nb or Ta at the surface of the catalyst, improving its catalytic performance. It has been shown that the undoped Mo-V-(Te and/or Sb)-O catalyst optionally containing Nb and/or Ta, presented a selectivity to acrylic acid lower than 40% while selectivities to acrylic acid of about 60% can be obtained on Nb or Ta-doped catalysts.

Thus, in a first aspect, the present invention provides a process for preparing an improved catalyst having the formula (I) :

$$Mo_1V_a(Te\text{ and/or }Sb)_b(Nb\text{ and/or }Ta)_cSi_dO_x \qquad (I)$$

Wherein a is between 0.006 and 1
b is between 0.006 and 1
c is between 0.001 and 0.5
d is between 0 and 3.5
and x is dependent on the oxidation state of the other elements,

said process comprising, in a first step, the synthesis of a mainly orthorhombic phase of a Mo-V-(Te and/or Sb)-O mixed metal oxide, optionally containing Nb and/or Ta and/or Si, followed in a second step, by the addition to the mixed metal oxide issued from the first step, of a doping agent constituted by at least one component selected from Nb and Ta

In a second aspect, the present invention provides catalysts obtainable by the process according to the first aspect of the invention.

In a third aspect, the present invention provides a process for producing acrylic acid which comprises subjecting propane to a vapour phase catalytic oxidation reaction in the presence of a catalyst produced by the process according to the first aspect of the invention.

The present invention is described in detail below.

Detailed description of the invention

The improved catalyst prepared by the process of the present invention has the empirical formula (I) :

$$Mo_1V_a(Te\text{ and/or }Sb)_b(Nb\text{ and/or }Ta)_cSi_dO_x \qquad (I)$$

Wherein a is between 0.006 and 1
b is between 0.006 and 1
c is between 0.001 and 0.5
d is between 0 and 3.5

and x is dependent on the oxidation state of the other elements.

Preferably, a is between 0.1 and 0.5

b is between 0.01 and 0.3
c is between 0.001 and 0.25
d is between 0 and 1.6
and x is dependent on the oxidation state of the other elements.

The amount of Nb and/or Ta, expressed by c, is the sum of the amount optionally present in the mixed metal oxide issued from the first step of the process (c') and the amount added during the second step of the process (c") :

Preferably, c' is between 0 and 0.15
c" is between 0.001 and 0.1

The preferred catalyst prepared by the process of the invention has the empirical formula

$$Mo_1V_aSb_bNb_cSi_dO_x \qquad (II)$$

Wherein a, b, c and d may vary in the ranges defined above.

In the first step of the process of the invention, the mainly orthorhombic phase of a Mo-V-(Te and/or Sb)-O mixed metal oxide, optionally also containing Nb and/or Ta and/or Si may be provided by hydrothermal reaction method (HTT) or by dry-up method (DRU).

The mixed metal oxides are generally characterized by their X-ray diffraction patterns. Many authors have described the phases that may be present, for example in: Applied Catalysis A:General 232 (2002) 77-92 ; Applied Catalysis A: General 244 (2003) 359-370 ; Catalysis Letters Vol. 74 N°3-4 (2001) 149-154 ; Catalysis Surveys from Japan Vol. 6, N°1/2 (October 2002) 33-44 ; Chem. Mater. (2003) Vol. 15, N°11, 2112-2114.

The X-ray patterns show mixtures of phases, such as orthorhombic, hexagonal or also $Mo_3O$ phases. Mainly orthorhombic phase in the present invention means that the mixed metal oxide comprises more than 50% by weight of orthorhombic phase in the crystallized solid, preferably more than 70% and more preferably more than 80%. The amount in orthorhombic phase may be determined after calibration with the pure phases, according to the method of washing and weighting described in the Japanese Laid-Open Patent application Publication N°10-330343 and the X-ray diffraction patterns as described in WO 2004/105938.

A wide range of starting materials including, for example, oxides, nitrates, halides or oxyhalides, alkoxides, acetylacetonates or organometallic compounds may be used.

For example, ammonium molybdate, ammonium paramolybdate or ammonium heptamolybdate may be used for the source of molybdenum in the catalyst. However, compounds such as $MoO_3$, $MoO_2$, $MoCl_5$, $MoOCl_4$, $Mo(OC_2H_5)_5$, molybdenum acetylacetonate, phosphomolybdic acid and silicomolybdic acid may also be utilized.

Similarly, ammonium metavanadate may be utilized for the source of vanadium in the catalyst. However, compounds such as $V_2O_5$, $V_2O_3$, $VOCl_3$, $VCl_4$, $VOSO_4$, $VO(C_2H_5)_3$, vanadium or vanadyl acetylacetonate may also be utilized.

The tellurium source may include telluric acid, $TeCl_4$, $Te(OC_2H_5)_5$, $Te(OCH(CH_3)_2)_4$ and $TeO_2$.

The antimony source may include antimony trioxide, $Sb_2(SO_4)_3$, $SbCl_3$ or $SbCl_5$.

The niobium source may include niobium hydrogen oxalate, ammonium niobium oxalate, $Nb_2O_5$, $NbCl_5$, niobic acid or $Nb(OC_2H_5)_5$, $Nb(O-nBu)_5$, niobium tartrate ...

The tantalum source may include tantalic acid, tantalum oxalate, $TaCl_5$ or $Ta_2O_5$

Optionally, colloidal silica or polysilicic acid may be used.

Hydrothermal reaction and dry-up methods are well known and are described for example in Applied Catalysis A : General 194-195 (2000) 479-485.

In the present invention, when a hydrothermal synthesis is carried out, an aqueous mixture containing Mo, V, Te and/or Sb metal ions in the appropriate atomic ratio is prepared by mixing solution of the possible starting materials described above in water. The temperature is generally of from 20°C to 100°C, preferably from 20°C to 80°C. Optionally, silica in the form of colloidal silica or polysilicic acid and/or niobium or tantalum source may be added to this solution.

A solution or a slurry is formed. After the solution or the slurry is well stirred, it is introduced for example into a stainless steel autoclave and a reaction is carried out at a temperature in the range of 130°C to 260°C for a duration of 24 to 72 hours. A temperature in the range of 150°C to 200°C is preferred. The reaction provides a black solid, which is washed and dried. Drying methods include, without limitation, vacuum drying, freeze drying, spray drying, rotary evaporation and air drying.

The solid may further be subjected to calcination. The calcination may be conducted in an oxygen-containing atmosphere or in the substantial absence of oxygen, e.g. in an inert atmosphere or in vacuum. Suitable examples of inert atmosphere include without limitation nitrogen, argon, xenon, helium or mixtures thereof. Preferably, the inert atmosphere is nitrogen. The oxygen containing atmosphere or the inert atmosphere may flow over the surface of the catalyst or may not flow thereover.

Preferably, calcinations under static air at a temperature in the range of 250°C to 350°C for at least 10 minutes and under nitrogen at a temperature in the range of 550°C to 700°C for about at least 1 hour are conducted. More particularly, calcination is carried out under static air at 320°C for at least 20 minutes and then under nitrogen at 600° for 2 hours.

The mixed metal oxide synthetized by hydrothermal method has orthorhombic and hexagonal phases, mainly orthorhombic phase. Further treatments such as for example washing with hydrogen peroxide, oxalic acid, nitric acid solutions may optionally be applied to increase the content in orthorhombic phase.

A dry-up synthesis by any suitable method known in the art may be also carried out in the first step of the process of the invention. A mixture of orthorhombic and hexagonal phases may be obtained. On the same manner than described above, calcinations may be conducted and further treatments may increase the content in orthorhombic phase.

Preferably, the synthesis of the mixed metal oxide in the first step of the process is carried out by hydrothermal reaction method. More preferably, the mixed metal oxide issued from the first step doesn't contain Nb nor Ta.

The solid issued from the first step may be crushed, in order to obtain shorter needles.

The second step of the process of the invention comprises the addition to the metal oxide issued from the first step of a doping agent constituted by at least one component selected from Nb and Ta.

The addition of the doping agent may be carried out by impregnation with solutions containing a source of Nb and/or Ta. The Nb or Ta solutions may be prepared with the sources of Nb or Ta described above, or they may be commercial solutions. An appropriate concentration of these solutions is used in order to obtain the appropriate atomic ratio in the doped solid.

Typically, a mixture of the mixed metal oxide issued from the first step with an impregnation solution is stirred at room temperature during about one hour. The impregnation may be also conducted under a slightly elevated temperature. The mixture is then dried by any suitable method known in the art as described above. Generally, it is dried at a temperature of 70°C to 100°C, preferably at about 80°C during at least 2 hours.

The addition of the doping agent may be carried out alternatively by physical mixed method, e.g. mixing or crushing a Mo-V-(Sb and/or Te)-O sample, optionally containing Nb and/or Ta and/or Si, with a solid Nb or Ta oxide or a mixture of Nb oxide and Ta oxide. The mixing time is typically 5 to 15 minutes. Any other suitable method known in the art to mix solids may be used.

Preferably, the addition of the doping agent is done by impregnation with solutions containing a source of Nb and/or Ta.

The doped mixed metal oxide issued from the second step of the process may be used as a final catalyst, but it may further be subjected to calcination. The calcination may be conducted in an oxygen-containing atmosphere or in the substantial absence of oxygen, e.g. in an inert atmosphere or in vacuum. Suitable examples of inert atmosphere include without limitation nitrogen, argon, xenon, helium or mixtures thereof. Preferably, the inert atmosphere is nitrogen. The oxygen containing atmosphere or the inert atmosphere may flow over the surface of the catalyst or may not flow thereover. The calcination is usually performed at a temperature of from 200°C to 700°C, preferably from 300°C to 600°C. The calcination is performed for from 1 hour to 4 hours, preferably for from 1 hour to 2 hours.

In one mode of operation, the calcination is performed in two stages. In the first stage, the solid is calcined in an oxidizing environment (e.g. air) at a temperature of from 200°C to 400°C, preferably from 250°C to 350°C for from 1 hour to 4 hours. In the second stage, the material issued from the first stage is calcined in an inert atmosphere at a temperature of from 400°C to 700°C , preferably, from 500°C to 600°C for from 1 hour to 2 hours.

Further calcination in static air and/or nitrogen flow provides a solid Nb-and/or Ta-doped Mo-V-(Sb and/or Te)-O catalyst.

One preferred process consists in preparing a Mo-V-Sb-O mixed oxide by hydrothermal method and activation in air and nitrogen, followed by impregnation with a niobium solution and calcination in air and nitrogen.

In a second aspect, the present invention provides catalysts obtainable by the process according to the first aspect of the invention.

The catalyst may be used by itself as a solid catalyst for the production of acrylic acid from propane, but may be formed into a catalyst together with a suitable carrier such as silica, alumina, titania, aluminosilicate, diatomaceous earth or zirconia. Further, it may be molded into a suitable shape and particle size depending upon the scale or system of the reactor.

In a third aspect, the present invention provides a process for producing acrylic acid which comprises subjecting propane to a vapour phase catalytic oxidation reaction in the presence of a catalyst produced by the process according to the first aspect of the invention.

As a starting material gas to be supplied to the reaction system, a gas mixture comprising a steam containing propane and a molecular oxygen containing gas is usually used. However, the steam containing propane and the oxygen containing gas may be alternately supplied to the reaction system.

It is preferred to employ a starting material gas, which contains steam.

Further, as a diluting gas, an inert gas such as nitrogen, argon, or helium may be supplied. The molar ratio propane : oxygen : diluting gas : ($H_2O$) in the starting material gas in generally 1 : 0.05-3 : 1 -10 : 1-10, preferably 1 : 0.05-2 : 1-10 : 1-10 and more preferably 1 : 0.1-1 : 1-5 : 1-5.

To incorporate molecular oxygen into the feed gas, such molecular oxygen may be pure oxygen gas. However, it is usually more economical to use an oxygen containing gas such as air. It is important that propane and oxygen concentrations in the feed gases be maintained at the appropriate levels to minimize or avoid entering a flammable regime within the reaction zone or especially at the outlet of the reactor zone. It is also possible to carry out the vapour phase catalytic reaction in the absence of molecular oxygen. In such a case, it is preferred to adopt a method wherein a part of the catalyst is appropriately sent to an oxidation regenerator to be regenerated, and then returned to the reaction zone for reuse. The regeneration method of the catalyst, such as described in WO 04/0246665 or WO 04/0246666 may be used.

The reaction system may be a fixed bed system or a fluidized bed system. However, a fluidized bed system may preferably be employed whereby it is easy to control the reaction temperature.

The process may be practiced in a single pass mode -only fresh feed is fed to the reactor-, or in a recycle mode -at least a portion of the reactor effluent is returned to the reactor-.

General conditions for the process are as follows: The reaction temperature can vary from 200 to 500°C, but is usually in the range of from 250 to 450°C, more preferably 350 400°C. The reaction can be conducted usually under atmospheric pressure, but may be conducted under a slightly elevated pressure or slightly reduced pressure. Typical pressures are in the range of from $1.01 \ 10^4$ to $1.01 \ 10^6$ Pa, preferably from $5.05 \ 10^4$ to $5.05 \ 10^5$. The average contact time with the catalyst can be from 0.01 to 90 seconds, preferably from 0.1 to 30 seconds.

When the oxidation reaction of propane is conducted by the process of the present invention, carbon monoxide, carbon dioxide, acetic acid, acetone... may be produced as by-products, in addition to acrylic acid and propylene. The catalyst operates efficiently, significantly avoiding undesirable reactions such as further oxidation and favouring the selective formation of acrylic acid.

The present invention will be explained below in more detail by reference to examples and comparative examples, but the invention should not be construed as being limited to these examples.

In the following examples, the conversion of propane and the selectivity to acrylic acid are represented by the following formulas:

$$\text{Conversion of propane (\%)} = \text{moles of consumed propane/moles of supplied propane} \times 100$$

$$\text{Selectivity to acrylic acid(\%)} = \text{moles of formed acrylic acid/moles of supplied propane} \times 100$$

EXAMPLES

Catalyst preparation

Example 1 : Preparation of Mo-V-Sb-O

The mixed metal oxide with preparative composition of Mo: V: Sb = 6: 2: 1 was prepared by hydrothermal method. First, an amount of 5.35 g of $(NH_4)_6Mo_7O_{24} \cdot 4H_2O$ (WAKO Chemicals, 99%) was dissolved in 20 ml of water at 80°C. Then, an amount of 1.34 g of $Sb_2(SO_4)_3$ (SOEKAWA Chemicals, 99%) were successively added to the aqueous solution. This suspension was stirred for 15 min. Finally, an aqueous solution of 2.63 g of $VOSO_4 \cdot nH_2O$ (MITSUWA Chemicals, assay 62%) in 10 ml of distilled water was added to the dark blue suspension, (not completely dissolved at this stage). After 15 min of stirring, the slurry was introduced in the Teflon inner tube in a stainless steel autoclave. The autoclave was sealed and heated at 175°C for 24 h. The black solid obtained was washed with distilled water and dried at 80 °C for 12 h. It was first calcined in static air at 320 °C for 20 min and then under nitrogen flow (50 ml/min) at 600 °C for 2 h.

2 batches of catalysts have been prepared. They will be called the MVS-S (S stands for Soekawa) or MVS-C (C stands for CBMM) samples depending on the following treatments. For the following impregnations, the samples were crushed for 5 minutes, in order to obtain shorter needles.

Example 2 : Preparation of Nb-doped Mo-V-Sb-O by niobium hydrogen oxalate (SOEKAWA Chem.)

Nb-doped Mo-V-Sb-O catalysts (with preparative Nb/Mo atomic ratios 0.008/6, 0.016/6, and 0.032/6) was prepared by impregnation (Volume of solution: 10 ml) of the MVS-S sample (1g) with colloidal solutions of Nb $(HC_2O_4)_5 \cdot nH_2O$ (SOEKAWA Chem., Assay $Nb_2O_5$ 14.9%). The sample was stirred for 1h at room temperature and dried at 80°C for 12h. It was first calcined in static air at 320 °C for 20 min and then under nitrogen flow (50 ml/min) at 600 °C for 2 h. After activation samples will be called the Nb-S-0.008, Nb-S-0.016 and Nb-S-0.032 sample, respectively.

Example 3 : Preparation of Nb-doped Mo-V-Sb-O by ammonium niobium oxalate (CBMM -Lot Number AD/3084)

Nb-doped Mo-V-Sb-O catalysts (with preparative Nb/Mo atomic ratios 0.032/6 and 0.064/6) were prepared by impregnation of the MVS-C catalyst sample (1g) with solutions (Volume 10ml) of $NH_4 [NbO(C_2O_4)_2(H_2O)_2] \cdot nH_2O$ (CBMM, Assay Nb 17.8%). The samples was(were ?) stirred for 1 h at room temperature and dried at 80°C for 12h. It was first calcined in static air at 320 °C for 20 min and then under nitrogen flow (50 ml/min) at 600 °C for 2 h. After activation samples will be called the Nb-C-0.032 and Nb-C-0.064 sample, respectively.

Example 4 : Preparation of Nb-doped Mo-V-Sb-O by physical mixed method

An amount of 0.1 g $Nb_2O_5$ powder (from WAKO) added to MVS-C sample (0.5g) and mixed for 5 min (while crushing in an agate mortar). It was calcined under nitrogen flow (50 ml/min) at 600 °C for 2 h. Catalyst weight was decreased from 0.6 to 0.56g. It will be called MVS-C-$Nb_2O_5$ sample.

Example 5 (comparative)

For comparative purpose, MVS-S catalyst sample was treated with water only and with a solution of 0.04M $(NH_4)_2C_2O_4$ (KATAYAMA Chemicals, 99.5%) in the same way as that described in example 2. These will be called MVS-$H_2O$ and MVS-AO (AO=Ammonium Oxalate), respectively.

Catalysts characterization and Activity test

Powder XRD patterns were recorded with a Rigaku Ris-Ivb diffractometer using Cu Ka radiation. Samples were ground and put on a horizontal sample holder. XRD patterns were recorded in the 2 to 60 ° range.
Propane oxidation was carried out at atmospheric pressure in a conventional flow system with a fixed-bed Pyrex tubular reactor in the temperature range of 300 to 380 °C. The feed composition was $C_3H_8$: $O_2$: $N_2$: $H_2O$ = 6.5: 10: 38.5: 45 vol. %. The amount of catalyst was 500 mg. The total flow rate was 20ml/min. Both reactants and products were analyzed by an on-line GC system equipped with the following columns: (1) Gaskuropack 54 to separate hydrocarbons and $CO_2$, (2) Molecular Sieve 13X to separate $O_2$, $N_2$ and CO and (3) Porapak QS to separate oxygenated products (acetone, acetic acid and acrylic acid). Blank runs showed that under the experimental conditions used in the present study, homogeneous gas-phase reaction was negligible.

Evaluation and Results

Example 6 :

Catalysts of example 1 (MVS-S and MVS-C sample), example 2 (Nb-S-0.008, Nb-S-0.016 and Nb-S-0.032 sample) and example 3 (Nb-C-0.032 and Nb-C-0.064 sample) were evaluated in propane selective oxidation to acrylic acid, as described above. The results are shown in table 1.

Table 1

| Catalyst | Reaction Temperature, °C | Conversion, % | | Selectivity, % | | | | | |
|---|---|---|---|---|---|---|---|---|---|
| | R.T. (°C) | $C_3H_8$ | $O_2$ | AA | PEN | Ace | AcA | CO | $CO_2$ |
| Example 1 MVS-S (comparative) | 300 | 15,8 | 26,6 | 35 | 13 | 5 | 18 | 15 | 14 |
| | 320 | 28,6 | 50,5 | 34 | 8 | 2 | 21 | 18 | 17 |
| | 340 | 41,7 | 82,5 | 27 | 6 | 1 | 22 | 22 | 22 |
| Example 2 Nb-S-0.008 | 300 | 18,2 | 27,1 | 45 | 13 | 5 | 15 | 11 | 11 |
| | 320 | 26,5 | 44,5 | 42 | 9 | 2 | 17 | 15 | 14 |
| | 340 | 39,0 | 73,9 | 33 | 6 | 1 | 19 | 21 | 20 |
| Example 2 Nb-S-0.016 | 280 | 8,5 | 11,7 | 42 | 22 | 12 | 11 | 7 | 7 |
| | 300 | 15,6 | 22,8 | 48 | 16 | 6 | 12 | 10 | 8 |
| | 320 | 24,5 | 37,3 | 49 | 11 | 3 | 14 | 12 | 10 |
| | 340 | 35,6 | 60,5 | 46 | 8 , | 1 | 15 | 15 | 14 |
| | 350 | 40,4 | 74,4 | 42 | 7 | 1 | 16 | 18 | 16 |
| | 360 | 45,7 | 87,8 | 37 | 8 | 1 | 16 | 20 | 18 |
| Example 2 Nb-S-0.032 | 280 | 5,4 | 7,2 | 45 | 28 | 12 | 6 | 4 | 5 |
| | 300 | 9,8 | 13,5 | 51 | 23 | 7 | 8 | 5 | 6 |
| | 320 | 14,8 | 22,1 | 57 | 17 | 4 | 9 | 7 | 6 |
| | 340 | 22,0 | 33,6 | 59 | 14 | 2 | 9 | 9 | 7 |
| | 360 | 29,0 | 47,3 | 58 | 11 | 1 | 10 | 10 | 9 |
| | 370 | 35,9 | 59,0 | 56 | 9 | 1 | 11 | 12 | 11 |
| | 380 | 42,3 | 74,4 | 52 | 7 | 1 | 11 | 16 | 14 |
| Example 1 MVS-C (comparative) | 280 | 8,2 | 12,8 | 36 | 15 | 15 | 16 | 8 | 9 |
| | 300 | 16,3 | 28,8 | 36 | 11 | 4 | 20 | 13 | 15 |
| | 320 | 23,9 | 43,6 | 35 | 9 | 3 | 21 | 15 | 17 |
| | 340 | 34,9 | 67,0 | 30 | 7 | 1 | 22 | 19 | 21 |
| Example 3 Nb-C-0.032 | 280 | 9,3 | 15,1 | 40 | 16 | 7 | 15 | 10 | 11 |
| | 300 | 14,9 | 25,3 | 43 | 13 | 4 | 16 | 11 | 13 |
| | 320 | 23,2 | 41,5 | 42 | 10 | 2 | 17 | 15 | 15 |
| | 340 | 33,8 | 63,8 | 38 | 7 | 1 | 18 | 17 | 18 |
| | 360 | 44,1 | 87,3 | 31 | 6 | 1 | 18 | 22 | 23 |
| Example 3 Nb-C-0.064 | 280 | 7,6 | 11,9 | 41 | 20 | 6 | 13 | 9 | 11 |
| | 300 | 14,1 | 24,8 | 45 | 15 | 3 | 15 | 10 | 13 |
| | 320 | 22,1 | 39,7 | 41 | 11 | 1 | 16 | 14 | 16 |
| | 340 | 31,2 | 58,6 | 37 | 9 | 1 | 16 | 18 | 19 |
| | 360 | 40,1 | 80,6 | 30 | 7 | 0 | 16 | 23 | 24 |

AA=acrylic acid, PEN=propylene, Ace=acetone, AcA=acetic acid

The results show that the impregnation treatment leads to an increase of selectivity in Acrylic acid but also for propylene. Over all selectivity in Acrylic Acid + Propylene (both valuable products) is increased with only a slight loss of conversion at the highest niobium loading. The activation energy for the conversion of propane is not affected by the impregnation treatment, indicating that niobium in this case does not interfere with propane activation. The improvement of selectivity in doped catalyst is then related to the reduced combustion or degradation of acrylic acid.

Example 7 :

The mechanical mixture of catalyst and niobium oxide of example 4 (MVS-C-$Nb_2O_5$ sample) was compared with a mechanical mixture of catalyst MVS-C and silicon carbide (well known to be inactive in the reaction). An increase of selectivity can be also observed in propane oxidation over MVS-C-$Nb_2O_5$, as it is shown in table 2.

Table 2

| Catalyst | Reaction Temperature, °C | Conversion, % | | Selectivity, % | | | | | |
|---|---|---|---|---|---|---|---|---|---|
| | R.T. (°C) | $C_3H_8$ | $O_2$ | AA | PEN | Ace | AcA | CO | $CO_2$ |
| Example 4 MVS-C-$Nb_2O_5$ | 280 | 6,4 | 12,3 | 39 | 21 | 6 | 13 | 9 | 12 |
| | 300 | 16,2 | 23,0 | 43 | 16 | 3 | 14 | 11 | 13 |
| | 320 | 22,1 | 35,4 | 44 | 12 | 2 | 16 | 11 | 15 |
| | 330 | 23,6 | 43,6 | 42 | 11 | 1 | 16 | 13 | 17 |
| | 350 | 35,6 | 66,4 | 38 | 8 | 1 | 17 | 17 | 20 |
| | | | | | | | | | |
| Comparative MVS-C-SiC- | 280 | 9,5 | 14,6 | 35 | 14 | 10 | 17 | 12 | 12 |
| | 300 | 16,8 | 26,3 | 37 | 11 | 6 | 20 | 12 | 14 |
| | 320 | 22,2 | 37,2 | 37 | 10 | 4 | 21 | 14 | 15 |
| | 340 | 31,4 | 57,8 | 32 | 8 | 2 | 22 | 18 | 19 |
| | 360 | 40,9 | 78,7 | 27 | 6 | 1 | 22 | 22 | 22 |

Example 8 :

In this example, catalysts of comparative example 5 (MVS-$H_2O$ and MVS-AO sample) were compared to catalyst Nb-S-0.016 of example 2 in propane selective oxidation to acrylic acid, as described above. The results are shown in table 3.

Table 3

| Catalyst | Reaction Temperature, °C | Conversion, % | | Selectivity, % | | | | | |
|---|---|---|---|---|---|---|---|---|---|
| | R.T. (°C) | $C_3H_8$ | $O_2$ | AA | PEN | Ace | AcA | CO | $CO_2$ |
| Example 5 MVS-H2O (comparative) | 280 | 10,9 | 18,7 | 36 | 14 | 7 | 17 | 12 | 14 |
| | 300 | 17,6 | 29,7 | 35 | 11 | 4 | 20 | 13 | 16 |
| | 320 | 25,2 | 45,8 | 33 | 9 | 2 | 21 | 16 | 19 |
| | 340 | 34,8 | 65,0 | 29 | 7 | 1 | 22 | 19 | 21 |
| | 360 | 42,6 | 83,9 | 24 | 6 | 1 | 22 | 22 | 25 |
| | | | | | | | | | |
| Example 5 MVS-AO (comparative) | 280 | 10,1 | 18,1 | 36 | 14 | 7 | 17 | 11 | 15 |
| | 300 | 16,3 | 31,4 | 35 | 10 | 4 | 19 | 15 | 17 |
| | 320 | 28,3 | 51,6 | 32 | 8 | 2 | 21 | 17 | 21 |
| | 340 | 35,5 | 72,3 | 27 | 6 | 1 | 22 | 19 | 24 |

(continued)

| Catalyst | Reaction Temperature, °C | Conversion, % | | Selectivity, % | | | | | |
|---|---|---|---|---|---|---|---|---|---|
| | R.T. (°C) | $C_3H_8$ | $O_2$ | AA | PEN | Ace | AcA | CO | $CO_2$ |
| | 350 | 41,4 | 83,2 | 24 | 6 | 1 | 22 | 21 | 26 |
| | | | | | | | | | |
| Example 2 Nb-S-0.016 | 280 | 8,5 | 11,7 | 42 | 22 | 12 | 11 | 7 | 7 |
| | 300 | 15,6 | 22,8 | 48 | 16 | 6 | 12 | 10 | 8 |
| | 320 | 24,5 | 37,3 | 49 | 11 | 3 | 14 | 12 | 10 |
| | 340 | 35,6 | 60,5 | 46 | 8 | 1 | 15 | 15 | 14 |
| | 350 | 40,4 | 74,4 | 42 | 7 | 1 | 16 | 18 | 16 |
| | 360 | 45,7 | 87,8 | 37 | 8 | 1 | 16 | 20 | 18 |

No improvement in selectivity to acrylic acid is recorded in the experiment with only water and with ammonium oxalate solution.

**Claims**

1. A process for preparing an improved catalyst having the formula (I) :

$$Mo_1V_a(Te\ and/or\ Sb)_b(Nb\ and/or\ Ta)_cSi_dO_x$$

Wherein a is between 0.006 and 1
b is between 0.006 and 1
c is between 0.001 and 0.5
d is between 0 and 3.5
and x is dependent on the oxidation state of the other elements,

said process comprising, in a first step, the synthesis of a mainly orthorhombic phase of a Mo-V-(Te and/or Sb)-O mixed metal oxide, optionally containing Nb and/or Ta and/or Si, followed in a second step, by the addition to the mixed metal oxide issued from the first step, of a doping agent constituted by at least one component selected from Nb and Ta

2. A process according to claim 1, wherein

a is between 0.1 and 0.5
b is between 0.01 and 0.3
c is between 0.001 and 0.25
d is between 0 and 1.6

3. A process according to claim 1 or 2, wherein c is the sum of c' and c", c' being the amount of Nb and/or Ta optionally present in the mixed metal oxide and c" the amount added during the second step, and

c' is between 0 and 0.15
c" is between 0.001 and 0.1

4. A process according to any claim 1 to 3, wherein the catalyst has the formula

$$Mo_1V_aSb_bNb_cSi_dO_x \qquad (II)$$

with a, b, c and d defined above.

5. A process according to any claim 1 to 4, wherein the mixed metal oxide issued from the first step is synthetized by hydrothermal reaction method (HTT) or by dry-up method (DRU)

6. A process according to any claim 1 to 5, wherein the mixed metal oxide issued from the first step is calcined.

7. A process according to any claim 1 to 6, wherein the mixed metal oxide issued from the first step is submitted to further treatments to increase the content in orthorhombic phase.

8. A process according to any claim 1 to 7, wherein the addition of the second step is carried out by impregnation with solutions containing a source of Nb and/or Ta.

9. A process according to any claim 1 to 7, wherein the addition of the second step is carried out by physical mixed method

10. A process according to any claim 1 to 9, wherein the product issued from the second step is calcined.

11. Catalysts obtainable by the process according to any claim 1 to 10

12. A process for producing acrylic acid which comprises subjecting propane to a vapour phase catalytic oxidation reaction in the presence of a catalyst produced by the process according to any claim 1 to 10.

**European Patent Office**

**EUROPEAN SEARCH REPORT**

Application Number

EP 05 29 0665

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | PATENT ABSTRACTS OF JAPAN vol. 1998, no. 06, 30 April 1998 (1998-04-30) & JP 10 028862 A (MITSUBISHI CHEM CORP), 3 February 1998 (1998-02-03) * abstract * | 1-12 | B01J23/00 B01J23/28 B01J23/20 B01J23/18 B01J37/02 B01J37/08 C07C51/215 C07C57/04 |
| X | -& JP 10 028862 A (MITSUBISHI CHEM CORP) 3 February 1998 (1998-02-03) * paragraphs [0005], [0007], [0008], [0010], [0012] - [0018] * ----- | 1-12 | |
| X | EP 0 997 454 A (MITSUBISHI CHEMICAL CORPORATION) 3 May 2000 (2000-05-03) * paragraphs [0014], [0015], [0026], [0027], [0031], [0032] * ----- | 1-12 | |
| D,X | EP 0 608 838 A (MITSUBISHI KASEI CORPORATION; MITSUBISHI CHEM IND; MITSUBISHI CHEMICAL) 3 August 1994 (1994-08-03) * the whole document * ----- | 1-12 | |
| | | | TECHNICAL FIELDS SEARCHED (Int.Cl.7) |
| A | EP 1 193 240 A (ROHM AND HAAS COMPANY) 3 April 2002 (2002-04-03) * paragraphs [0082], [0083], [0088] * ----- | 1 | B01J C07C |

-/--

The present search report has been drawn up for all claims

| Place of search | Date of completion of the search | Examiner |
|---|---|---|
| Munich | 19 July 2005 | Gosselin, D |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**EP 1 704 919 A1**

European Patent Office

**EUROPEAN SEARCH REPORT**

Application Number

EP 05 29 0665

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | VITRY D ET AL: "Mo-V-Te-(Nb)-O mixed metal oxides prepared by hydrothermal synthesis for catalytic selective oxidations of propane and propene to acrylic acid" APPLIED CATALYSIS A: GENERAL, ELSEVIER SCIENCE, AMSTERDAM, NL, vol. 251, no. 2, 30 September 2003 (2003-09-30), pages 411-424, XP004458147 ISSN: 0926-860X more particularly: page 413-414 "3.1 Hydrothermal synthesis of orthorhomic structure", page 416 "3.4. Catalytic performance of propane selective oxidation" and page 423 "5. Conclusions" * the whole document * ----- | 1-12 | |
| | | | TECHNICAL FIELDS SEARCHED (Int.Cl.7) |

The present search report has been drawn up for all claims

| Place of search | Date of completion of the search | Examiner |
|---|---|---|
| Munich | 19 July 2005 | Gosselin, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

13

**ANNEX TO THE EUROPEAN SEARCH REPORT
ON EUROPEAN PATENT APPLICATION NO.**                     EP 05 29 0665

This annex lists the patent family members relating to the patent documents cited in the above-mentioned European search report.
The members are as contained in the European Patent Office EDP file on
The European Patent Office is in no way liable for these particulars which are merely given for the purpose of information.

19-07-2005

| Patent document cited in search report | | | Publication date | Patent family member(s) | | Publication date |
|---|---|---|---|---|---|---|
| JP 10028862 | A | | 03-02-1998 | NONE | | |
| EP 0997454 | A | | 03-05-2000 | AU | 734073 B2 | 31-05-2001 |
| | | | | AU | 8130698 A | 10-02-1999 |
| | | | | CA | 2296675 A1 | 28-01-1999 |
| | | | | DE | 69818268 D1 | 23-10-2003 |
| | | | | DE | 69818268 T2 | 24-06-2004 |
| | | | | EP | 0997454 A1 | 03-05-2000 |
| | | | | US | 6294685 B1 | 25-09-2001 |
| | | | | ID | 24821 A | 24-08-2000 |
| | | | | WO | 9903825 A1 | 28-01-1999 |
| EP 0608838 | A | | 03-08-1994 | JP | 3237314 B2 | 10-12-2001 |
| | | | | JP | 7010801 A | 13-01-1995 |
| | | | | JP | 3334296 B2 | 15-10-2002 |
| | | | | JP | 6279351 A | 04-10-1994 |
| | | | | DE | 69402567 D1 | 22-05-1997 |
| | | | | DE | 69402567 T2 | 27-11-1997 |
| | | | | EP | 0608838 A2 | 03-08-1994 |
| | | | | US | 5380933 A | 10-01-1995 |
| EP 1193240 | A | | 03-04-2002 | BR | 0104325 A | 04-06-2002 |
| | | | | CN | 1347866 A | 08-05-2002 |
| | | | | EP | 1193240 A1 | 03-04-2002 |
| | | | | JP | 2002161065 A | 04-06-2002 |
| | | | | MX | PA01009748 A | 15-04-2002 |
| | | | | US | 2002123647 A1 | 05-09-2002 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 608838 A
- JP 10330343 A
- US 6060422 A
- US 20030013904 A
- US 200201831198 A
- WO 2004105938 A
- WO 040246665 A
- WO 040246666 A

**Non-patent literature cited in the description**

- *Applied Catalysis A:General,* 2002, vol. 232, 77-92
- *Applied Catalysis A: General,* 2003, vol. 244, 359-370
- *Catalysis Letters,* 2001, vol. 74 (3-4), 149-154
- *Catalysis Surveys from Japan,* October 2002, vol. 6 (1/2), 33-44
- *Chem. Mater.,* 2003, vol. 15 (11), 2112-2114
- *Applied Catalysis A : General,* 2000, vol. 194-195, 479-485